# EUROPEAN PATENT APPLICATION

(11) **EP 4 218 836 A1**
(43) Date of publication of application: **02.08.2023**
(21) Application number: 21871998.7
(22) Date of filing: 03.08.2021
(51) Int. Cl.: A61L 2/18, A61J 1/00, A61L 2/025, B65B 55/04, B65B 55/10

(54) **CONTINUOUS DECONTAMINATION DEVICE**

(30) Priority: 24.09.2020 JP 2020159277
(71) Applicant: Airex Co., Ltd., Nagoya-shi Aichi 453-0015 (JP)
(72) Inventor: KAWASAKI, Koji, Nagoya-shi Aichi 453-0015 (JP); KAKUDA, Daisuke, Nagoya-shi Aichi 453-0015 (JP); OGATA, Yoshitaka, Nagoya-shi Aichi 453-0015 (JP)
(74) Representative: Lederer & Keller Patentanwälte Partnerschaft mbB
(86) International application number: PCT/JP2021/028774
(87) International publication number: WO 2022/064866

(57) **Abstract**

The present invention provides a continuous decontamination device capable of achieving short-term operations to provide uniform decontamination levels at each part by employing decontamination agents such as hydrogen peroxide that have recently been widely used without using expensive electron accelerators and of treating articles to be decontaminated in large quantities.

The continuous decontamination device is provided with a device body composed of a decontamination region and an aeration region, a conveyance means for conveying an article, a mist supply means, and an aeration means. The conveyance means changes a part for supporting the article inside the decontamination region when the conveyance means supports the article carried from an inlet and conveys the article to an outlet. All external surfaces of the article can thus be decontaminated. The mist supply means includes an ultrasonic atomizer for converting a decontamination agent into a decontamination agent mist to concentrate the decontamination agent mist on external surfaces of the article conveyed inside the decontamination region. The aeration means removes with clean gas the decontamination agent mist that is residual on the external surfaces of the article.

## Description

### TECHNICAL FIELD

The present invention relates to a continuous decontamination device for continuously decontaminating external surfaces of an article with a decontamination agent mist and conveying the decontaminated article to a working chamber in a sterile environment.

### BACKGROUND ART

The convenience of the clinical environment allows for the production of pre-filled syringes and pre-filled vials filled with a pharmaceutical product. Each pharmaceutical product is filled in these syringes or vials in a filling working chamber in a sterile environment (hereinafter referred to as a "sterile working chamber"). Each of the syringes and vials used in this operation is small in size, and such tools to be treated are needed in large numbers. Then, these syringes and vials are sterilized by γ-ray irradiation, electron beam irradiation, EOG (ethylene oxide gas), and other approaches at each manufacturing stage, and carried in a sterile working chamber with a predetermined number thereof integrally accommodated in a package.

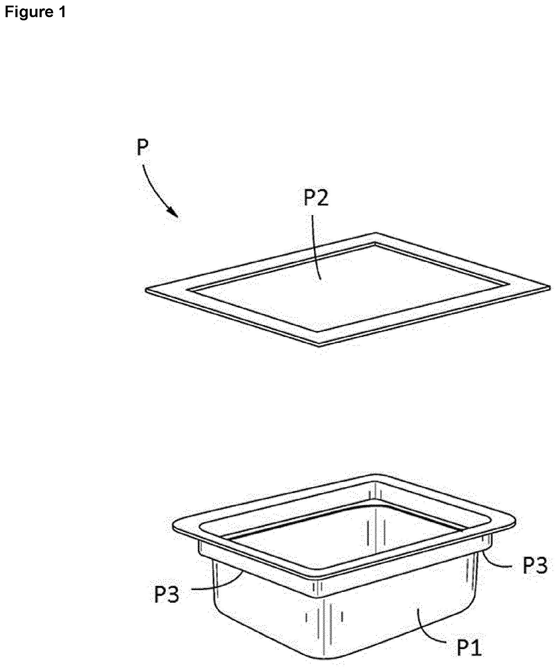

Illustrative example of the package includes a medical appliance package proposed in the following patent document 1 and disclosed as prior art ("P" in FIG. 1). Such a package is generally referred to as "peel-open package", and includes a plastic tab (P1 in FIG. 1) molded according to the shape of a medical appliance such as a syringe or vial accommodated therein and a gas-permeable upper surface seal (P2 in FIG. 1). The upper surface seal used is generally Tyvek (trademark) as a non-woven fabric composed of high-density polyethylene microfibers, and the Tyvek (trademark) is gas-permeable through micropores included in the Tyvek (trademark) product to the inside of the plastic tab, but blocks ingress of microorganisms.

The package thus configured is further packed on the exterior with a packaging bag to be circulated and conveyed. Unfortunately, when the package is circulated or conveyed, or taken out of the packaging bag to carry the same in a sterile working chamber, external surfaces of the plastic tab and the upper surface seal are contaminated. Therefore, such a contaminated package is not allowed to be carried in the sterile working chamber unless the external surfaces are decontaminated. Then, after the external surfaces of the plastic tab and the upper surface seal are decontaminated by a decontamination device connected to the sterile working chamber, the package is conveyed to the sterile working chamber, the upper surface seal is peeled open from the plastic tab in the sterile working chamber, and the filling into an internal sterilized syringe or vial is performed.

Generally, various methods using EOG (ethylene oxide gas), hydrogen peroxide (in the form of a gas or mist), ozone gas, plasma, γ-ray irradiation, ultraviolet irradiation or electron beam irradiation are introduced for each intended use in a decontamination device for decontaminating an accommodated body carried in the sterile working chamber. One of these methods is the most common method using hydrogen peroxide (in the form of a gas or mist). Advantageously, hydrogen peroxide has a strong sterilization effect, and is inexpensively available and effectively utilized as an environmentally-friendly decontamination gas that is ultimately decomposed into oxygen and water. However, the method using hydrogen peroxide is problematic due to longer duration required to obtain a necessary level of decontamination effect. Also, the hydrogen peroxide method unfortunately brings about longer duration required in the aeration operation for removing a condensed film of a hydrogen peroxide solution on the surface of the accommodated body after decontamination.

Meanwhile, in decontamination devices where numerous accommodated bodies are required to be treated per unit time, as in the production of pre-filled syringes, short-time treatment methods with a high decontamination effect are preferable. The following Non-Patent Document 1 describes a safe decontamination device integrated with a low-energy electron accelerator for obtaining a high decontamination effect as opposed to general devices using a decontamination agent such as hydrogen peroxide, and for providing high productivity and no residual material.

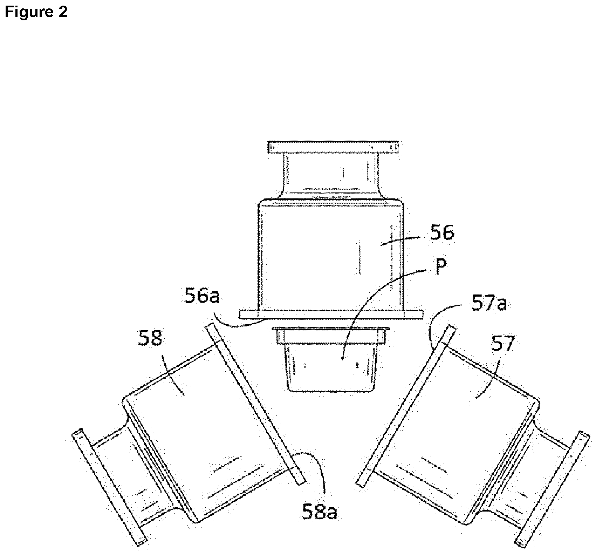

In fact, the decontamination device is operated to treat a package that accommodates pre-filled syringes, and the package including a prior-decontaminated syringe is conveyed to a sterile working chamber by a conveyor after external surfaces thereof are decontaminated with electron beams. The device, composed of 3 low-energy electron accelerators (56, 57, 58 in FIG. 2) arranged at an angle of 120 degrees with each other, irradiates with electron beams all the surfaces of the package through irradiation windows (56a, 57a, 58a) in 3 directions.

In such a device, a plastic tab and an upper surface seal can efficiently be decontaminated by controlling the dose of the electron beams for irradiation. The following Non-Patent Document 1 describes that this device can treat as many as 3,600 syringes per hour and achieve high productivity.

### CITATION LIST

### PATENT LITERATURE

Patent Document 1: JP-A-4237489

### NON-PATENT LITERATURE

Non-Patent Document 1: Radiation Application Technology Database, Data Number: 010306 (Prepared by Masayuki Sekiguchi on October 3, 2007), Radiation Application Development Association

### SUMMARY OF INVENTION

### TECHNICAL PROBLEM

In the decontamination device in the above Non-Patent Document 1, 3 low-energy electron accelerators, arranged at an angle of 120 degrees with each other on an outer peripheral side of a medical appliance package conveyed in the conveyance direction, irradiate with electron beams at the same time the entire external surfaces of the medical appliance package for decontamination (see FIG. 2).

This method is applicable enough to irradiate with electron beams external surfaces of a medical appliance package (upper surface portion, bottom surface portion and right and left side surface portions). However, this device is insufficient to irradiate with electron beams in that front and rear side surface portions are located apart in the conveyance direction of the medical appliance package. Therefore, it is difficult to maintain a high level of reliability and safety of such a decontamination effect. Thus, the irradiation intensity is required to be higher by making larger irradiation windows of each electron accelerator produced, controlling the irradiation angle and increasing the accelerating voltage of each electron accelerator because the package is distant from the irradiation windows of each electron accelerator when front and rear side surface portions of the medical appliance package are irradiated with electron beams from an outer peripheral portion.

Generally, each low-energy electron accelerator, having a large irradiation area and a potentially higher accelerating voltage, is expensive. In addition, the use limit (service life) of an electron accelerator due to its integrated time in use is shorter when the accelerating voltage is set to be high, resulting in higher maintenance costs by replacement. Therefore, the initial costs and maintenance costs of the device are unfortunately higher due to simultaneous operation of the 3 expensive devices.

Meanwhile, the irradiation intensity varies depending on parts of a medical appliance package, and thus parts close to irradiation windows of an electron accelerator are irradiated with excessive electron beams to cause damage to the medical appliance package when the irradiation intensity of each electron accelerator is set higher to sufficiently decontaminate the front and rear side surface portions of the medical appliance package. In addition, what is problematic is a different decontamination level of each part because the distance between each part of the medical appliance package and irradiation windows of each electron accelerator varies.

Thus, methods using a decontamination agent such as hydrogen peroxide that have recently been used widely are problematic because the decontamination of a medical appliance package requires treatment for a long period of time and in large quantities while they provide strong decontamination effects, low expenses and environmental friendliness. Meanwhile, the method using an electron accelerator unfortunately causes high device prices and maintenance costs and different decontamination levels at each part, while medical appliance packages to inevitably be treated in large quantities can effectively be decontaminated.

Thus, the present invention was made in view of the situation to solve the problems, and has an object to provide a continuous decontamination device capable of achieving short-term operations to provide uniform decontamination levels at each part by employing decontamination agents such as hydrogen peroxide that have recently been widely used without using expensive electron accelerators and of treating articles to be decontaminated in large quantities.

### SOLUTION TO PROBLEM

To solve the aforementioned problem, inventors of the present invention have carried out an extended investigation to find that an ultrasonic atomizer converts a decontamination agent such as a hydrogen peroxide solution into a fine decontamination agent mist to allow the decontamination agent mist to concentrate on the surface of an article to be decontaminated in a decontamination device. Based on that technique, the present invention was accomplished.

Specifically, a continuous decontamination device according to the present invention is, according to description in claim 1,
a continuous decontamination device (10, 110) connected to a sterile working chamber (20, 120) for decontaminating external surfaces of an article (P) with a decontamination agent mist (41, 141) and conveying the article to the inside of the sterile working chamber, the continuous decontamination device including:
a device body (10a, 110a) composed of a decontamination region (11, 111) and an aeration region (12, 112); a conveyance means (30, 130) for conveying an article; a mist supply means (mist discharging device); and an aeration means (50, 150), characterized in that
the device body includes an inlet (13, 113) for carrying the article before decontamination in the decontamination region and an outlet (14, 114) for carrying the decontaminated article out of the aeration region,
the conveyance means can decontaminate all external surfaces of the article by changing a part for supporting the article inside the decontamination region when the conveyance means supports the article carried from the inlet and conveys the article to the outlet through the inside of the decontamination region and the aeration region,
the mist supply means includes an ultrasonic atomizer for converting a decontamination agent into the decontamination agent mist and supplying the same to the inside of the decontamination region to concentrate the decontamination agent mist on external surfaces of the article conveyed inside the decontamination region by the conveyance means, and
the aeration means removes with clean gas the decontamination agent mist that is residual on the external surfaces of the article conveyed by the conveyance means from the decontamination region.

Moreover, the present invention is, according to description in claim 2, the continuous decontamination device (10) according to claim 1, characterized in that
the conveyance means (30) includes an article conveyance device (31a, 31b, 31c, 31d) for conveying an article inside the decontamination region (11) and the aeration region (12) and a support changing device (32) for changing a part for supporting the article, characterized in that
the article conveyance device continuously conveys a plurality of articles carried from the inlet in either an elevated or a lowered direction inside the decontamination region and the aeration region to carry the articles out of the outlet, and
the support changing device removes the articles from the article conveyance device inside the decontamination region and supports the articles on the article conveyance device again.

Furthermore, the present invention is, according to description in claim 3, the continuous decontamination device (110) according to claim 1, characterized in that
the conveyance means (130) includes an article conveyance device (131a, 131b, 131c) for conveying an article inside the decontamination region (111) and the aeration region (112) and a support changing device (132) for changing a part for supporting the articles, characterized in that
the article conveyance device continuously conveys a plurality of articles carried from the inlet in the horizontal direction inside the decontamination region and the aeration region to carry the articles out of the outlet, and
the support changing device removes the articles from the article conveyance device inside the decontamination region and supports the articles on the article conveyance device again.

Moreover, the present invention is, according to description in claim 4, the continuous decontamination device according to any one of claims 1 to 3, characterized in that
the article to be decontaminated is an accommodated body for accommodating a medical appliance such as a sterile syringe or a vial.

### ADVANTAGEOUS EFFECTS OF INVENTION

According to the above configuration, a continuous decontamination device according to the present invention includes a device body composed of a decontamination region and an aeration region, a conveyance means for conveying an article, a mist supply means, and an aeration means. The device body includes an inlet for carrying an article before decontamination in the decontamination region and an outlet for carrying the decontaminated article out of the aeration region. The conveyance means changes a part for supporting the article inside the decontamination region when the conveyance means supports the article carried from the inlet and conveys the article to the outlet through the inside of the decontamination region and the aeration. Accordingly, all external surfaces of the article can be decontaminated.

The mist supply means includes an ultrasonic atomizer for converting a decontamination agent into a decontamination agent mist and supplying the same to the inside of the decontamination region. The mist supply means concentrates a decontamination agent mist on external surfaces of the article conveyed inside the decontamination region by the conveyance means. The aeration means removes with clean gas the decontamination agent mist that is residual on the external surfaces of the article conveyed from the decontamination region by the conveyance means.

Thus, according to the above configuration, the present invention can provide a continuous decontamination device capable of achieving short-term operations to provide uniform decontamination levels at each part by employing decontamination agents such as hydrogen peroxide that have recently been widely used without using expensive electron accelerators and of treating articles to be decontaminated in large quantities.

According to the above configuration, the conveyance means includes an article conveyance device for conveying an article inside the decontamination region and the aeration region and a support changing device for changing a part for supporting the article. The article conveyance device continuously conveys a plurality of articles carried from the inlet in either an elevated or a lowered direction inside the decontamination region and the aeration region to carry the articles out of the outlet. The support changing device removes the articles from the article conveyance device inside the decontamination region and supports the articles on the article conveyance device again. Accordingly, the above operational advantage can more specifically be provided.

According to the above configuration, the conveyance means includes an article conveyance device for conveying an article inside the decontamination region and the aeration region and a support changing device for changing a part for supporting the article. The article conveyance device continuously conveys a plurality of articles carried from the inlet in the horizontal direction inside the decontamination region and the aeration region to carry the articles out of the outlet. The support changing device removes the articles from the article conveyance device inside the decontamination region and supports the articles on the article conveyance device again. Accordingly, the above operational advantage can more specifically be provided.

According to the above configuration, the article to be decontaminated may be an accommodated body for accommodating a medical appliance such as sterile syringes and vials. Accordingly, the above operational advantage can more specifically be provided.

### BRIEF DESCRIPTION OF DRAWINGS

FIG. 1 is a perspective view illustrating an accommodated body to be decontaminated (package) in continuous decontamination devices according to first and second embodiments;
FIG. 2 is a schematic diagram illustrating the arrangement of an electron accelerator of a continuous decontamination device of a non-patent document 1;
FIG. 3 is a schematic cross-sectional view showing the inside of a vertical continuous decontamination device according to the first embodiment seen in a front view;
FIG. 4(A) is a front view showing the state where a rolling conveyor of the first embodiment supports a package P, and FIG. 4(B) is a side view thereof;
FIG. 5(C) is a front view showing the state where the support of the package P is changed from the rolling conveyor in FIG. 4 by a support changing device and FIG. 5(D) is a side view thereof;
FIG. 6 is a schematic cross-sectional view showing the inside of a horizontal continuous decontamination device according to the second embodiment seen in a front view;
FIG. 7(A) is a front view showing the state where a hanging conveyor of the second embodiment supports the package P, and FIG. 7(B) is a side view thereof;
FIG. 8(C) is a front view showing the state where the support of the package P is changed from the hanging conveyor in FIG. 8 by the support changing device, and FIG. 8(D) is a side view thereof; and
FIG. 9(A) is a front view showing the state where a support catch supports the package P in place of a support bar as an alternative of each of the embodiments, and FIG. 9(B) is a side view thereof.

### DESCRIPTION OF EMBODIMENTS

In the present invention, "mist" is broadly interpreted as the state of a liquid droplet of a decontamination agent refined and floating in the air, the state of a gas and a liquid agent of a decontamination agent in mixture, the state of the decontamination agent to repeat the change in phase between condensation and evaporation of a gas and a droplet, and the like. In terms of particle size as well, the mist is also broadly interpreted to include mists (the size may be defined as 10 µm or less), fogs (the size may be defined as 5 µm or less), and liquid droplets, which can be subclassified. In the present invention, an ultrasonic atomizer converts a mist, a fog or a liquid droplet into an equalized ultrafine particle to provide high-level decontamination effects even in a short period of time.

A continuous decontamination device according to the present invention will be described with reference to each embodiment. The present invention is not restricted to each of the following embodiments. In the continuous decontamination device according to each of the following embodiments, a decontamination agent used is hydrogen peroxide. First, an article to be decontaminated by hydrogen peroxide will be described. In each embodiment, such an article to be decontaminated is an accommodated body (package) for accommodating a medical appliance such as a syringe and a vial. In this invention, the article to be decontaminated is not restricted to such an accommodated body (package), and it may be used so long as it is continuously decontaminated and conveyed to a sterile working chamber.

### <First embodiment>

FIG. 1 is a perspective view illustrating an accommodated body to be decontaminated (package) in a continuous decontamination device according to a first embodiment. Nevertheless, in the present invention, the shape of the accommodated body is not restricted to that in FIG. 1 only. In FIG. 1, a package P includes a polyethylene tab P1 and an upper surface seal P2 of Tyvek (trademark). In this first embodiment, numerous sterilized syringes used for filling a pre-filled syringe therein are accommodated and decontaminated in a sealed manner.

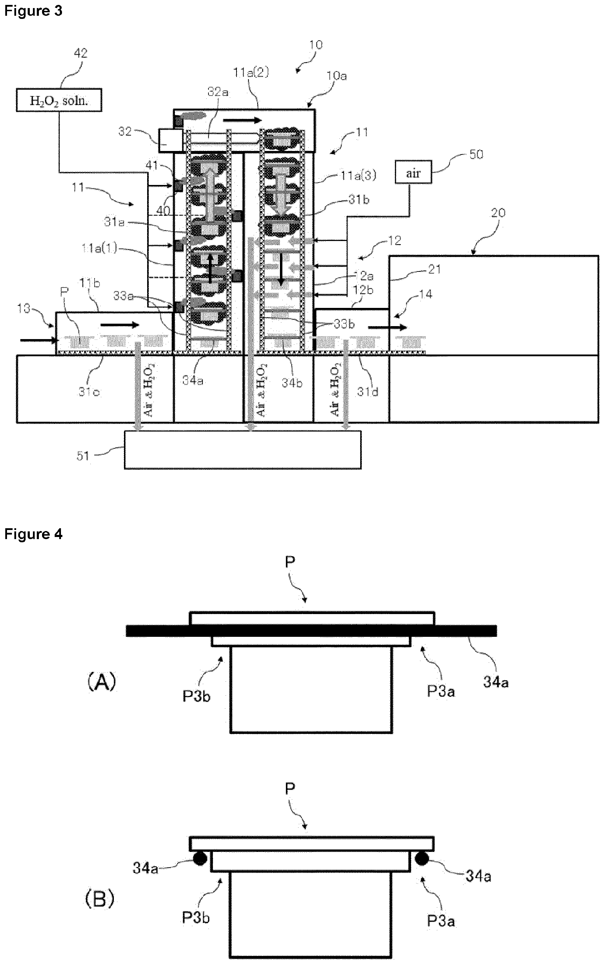

Herein, the continuous decontamination device of this first embodiment will be described. FIG. 3 is a schematic cross-sectional view showing the inside of a vertical continuous decontamination device according to the first embodiment seen in a front view. The continuous decontamination device according to this first embodiment includes a device body composed of a decontamination region and an aeration region, a conveyance device for conveying a package P, a mist supply device, and an aeration device (an air supply and exhaust device of clean air for aeration). The continuous decontamination device according to this first embodiment is a vertical continuous decontamination device for conveying a package P in elevated and lowered directions in the decontamination region and the aeration region.

In FIG. 3, a device body 10a of a continuous decontamination device 10 according to this first embodiment is covered with an outer wall portion made of stainless metal plate on the periphery, linked to a side wall 21 of an isolator 20, and placed on the floor. The device body 10a is divided into a decontamination region 11 and an aeration region 12. The decontamination region 11 is divided into a decontamination region body 11a and an introduction region 11b. One wall portion of the introduction region 11b is provided with an inlet 13 for carrying the package P to the inside of the continuous decontamination device 10. Meanwhile, the aeration region 12 is divided into an aeration region body 12a and a delivery region 12b. One wall portion of the delivery region 12b is provided with an outlet 14 for carrying the package P out of the device, which communicates with the side wall 21 of the isolator 20.

In this first embodiment, a portion of the decontamination region body 11a of the device body 10a and a portion of the aeration region body 12a are allowed to change the device volume (region's length). Specifically, in FIG. 3, the decontamination region body 11a is configured by a decontamination region first portion 11a (1) that is contact with the introduction region 11b (leftward in the figure), a decontamination region second portion 11a (2) of an upper portion of the device (upward in the figure) and a decontamination region third portion 11a (3) that is contact with the aeration region body 12a (upper right half in the figure). On the other hand, the aeration region body 12a is configured by a section defined between the decontamination region third portion 11a (3) and the delivery region 12b.

Thus, the reason for a larger device volume of the decontamination region body 11a than the aeration region body 12a is as follows. In the present invention, while fine hydrogen peroxide solution mists are efficiently utilized to assuredly achieve uniform decontamination levels, the use of hydrogen peroxide solution mists in small amounts improves the aeration efficiency. Thus, despite the resulting larger device volume of the decontamination region body 11a than the aeration region body 12a, an article to be decontaminated can be treated in large quantities even if the total volume is equivalent to or less than those of conventional devices.

A conveyance device 30 for conveying a package P is disposed inside the device body 10a. The conveyance device 30 is configured by an article conveyance device 31 and a support changing device 32. The article conveyance device 31 includes a rolling conveyor 31a for conveying a package P in an elevated direction (from a bottom portion to a top portion) from the decontamination region first portion 11a(1) to the decontamination region second portion 11a(2) and a rolling conveyor 31b for conveying the package P in a lowered direction (from the top portion to the bottom portion) inside the aeration region body 12a from the decontamination region second portion 11a(2) through the decontamination region third portion 11a(3).

Furthermore, the article conveyance device 31 includes a roller conveyor 31c for conveying a package P from the inlet 13 of the introduction region 11b to the bottom portion of the decontamination region first portion 11a (1) and a roller conveyor 31d for conveying the package P from the bottom portion of the aeration region body 12a to the outlet 14 of the delivery region 12b. Meanwhile, the support changing device 32 is provided at the decontamination region second portion 11a (2) to remove the package P from the top portion of the rolling conveyor 31a and support the package P at the top portion of the rolling conveyor 31b. In the present invention, the operational mechanism of the support changing device 32 is not particularly restricted. For example, such an operational mechanism may be a pusher for pushing a package P or a gripper for gripping the package P for replacement. In this first embodiment, a pusher 32 is employed. The operation of the pusher 32 will be described later.

The type of a conveyance device for conveying a package P is not particularly restricted. Such a conveyance device may be a combination of a roller conveyor for conveying an article on which a bottom wall surface thereof is placed, a conveyor device such as a mesh conveyor, and a support device such as a timing belt for conveying by supporting a side surface portion, a timing lift, a rolling conveyor and a shuttle conveyor.

Herein, the structure and operation of a rolling conveyor employed in this first embodiment will be described. The rolling conveyors 31a, 31b have the same structure, and here the structure of the rolling conveyor 31a will be described. In FIG. 3, the rolling conveyor 31a includes 2 sets of carriers having the same structure to support a package P on both side surfaces. FIG. 3 illustrates one carrier only and no other carrier hidden on its back side.

Each of the carriers is configured by two drive shafts 33a and a plurality of support bars 34a arranged over these drive shafts (see FIG. 3). The two drive shafts 33a are each composed of a loop-shaped chain disposed in the vertical direction from the decontamination region first portion 11a (1) to the decontamination region second portion 11a (2), which is revolved in the vertical direction by a drive mechanism (not shown). Thus, as the two drive shafts 33a are revolved in the vertical direction, the plurality of support bars 34a is revolved along the shafts in the vertical direction.

In rotation, the plurality of support bars 34a conveys the package P in the elevated direction (from the bottom portion to the top portion) inside the decontamination region first portion 11a (1) by supporting the package P on both side surfaces. The rolling conveyor 31b having the same structure conveys the package P in the lowered direction (from the top portion to the bottom portion) inside the decontamination region third portion 11a (3) and the aeration region body 12a by allowing the drive mechanism to rotate the package P in the reverse direction.

Subsequently, the state where the support bar 34a supports the package P on both side surfaces will be described. FIG. 4(A) is a front view showing the state where the rolling conveyor 31a of the first embodiment supports the package P, and FIG. 4(B) is a side view thereof. FIG. 4 illustrates a support bar 34a only and no drive shaft 33a. In FIG. 4, the support bar 34a supports a first shoulder portion P3a of the package P on both side surfaces. In this state, the package P is conveyed by the rolling conveyor 31a in the elevated direction (from the bottom portion to the top portion) inside the decontamination region first portion 11a (1) (see FIG. 3). The package P includes a second shoulder portion P3b below the first shoulder portion P3a. The operation of the second shoulder portion P3b will be described later.

In such a configuration, the inside of the continuous decontamination device 10 and decontamination operations will be described. In FIG. 3, an operator (not shown) who stays in the external environment places a plurality of packages P on the roller conveyor 31c for driving. The packages P are carried to the inside of the introduction region 11b through the inlet 13 of the introduction region 11b, while being placed on the roller conveyor 31c for driving.

Subsequently, the packages P are carried to the inside of the decontamination region first portion 11a (1) and supported by the rolling conveyor 31a. Herein, the roller conveyor 31c and the rolling conveyor 31a are alternately operated in an intermittent manner. Specifically, the roller conveyor 31c is operated with the rolling conveyor 31a stopped to convey and stop the packages P to the bottom portion of the rolling conveyor 31a. Subsequently, the rolling conveyor 31a is operated with the roller conveyor 31c stopped, and the packages P are conveyed by one step in the elevated direction inside the decontamination region first portion 11a (1) and stopped while the first shoulder portion P3a is supported on both side surfaces by the support bar 34a of the rolling conveyor 31a. Thus, the roller conveyor 31c and the rolling conveyor 31a are alternately operated in an intermittent manner to convey the packages P from the bottom portion to the top portion of the decontamination region first portion 11a (1).

A plurality of mist supply devices 40 (6 devices in FIG. 3) is disposed on side wall surfaces of the decontamination region first portion 11a (1) and the decontamination region second portion 11a (2) to discharge from the side surface a hydrogen peroxide solution mist 41 toward the packages P conveyed by the rolling conveyor 31a. As a result, the hydrogen peroxide solution mist 41 is uniformly filled entirely inside the decontamination region body 11a to continuously decontaminate the plurality of packages P being conveyed. The mist supply devices 40 will be described later.

Accordingly, the packages P decontaminated inside the decontamination region body 11a are carried to the inside of the decontamination region second portion 11a (2) through the top portion of the decontamination region first portion 11a (1). Herein, the pusher 32 is operated to remove the packages P from the top portion of the rolling conveyor 31a and support the packages P at the top portion of the rolling conveyor 31b.

Herein, the operation of the pusher 32 will be described. In FIG. 3, the pusher 32 includes a cylinder 32a for expanding and contracting in the horizontal direction inside the decontamination region second portion 11a (2). The packages P at the top portion of the rolling conveyor 31a are pushed by allowing the cylinder 32a of the pusher 32 to expand and removed by sliding from the top portion of the rolling conveyor 31a. Subsequently, the packages P are supported at the top portion of the other rolling conveyor 31b by allowing the cylinder 32a to further expand. In the state where the pusher 32 is operated, both the rolling conveyor 31a and the rolling conveyor 31b are stopped.

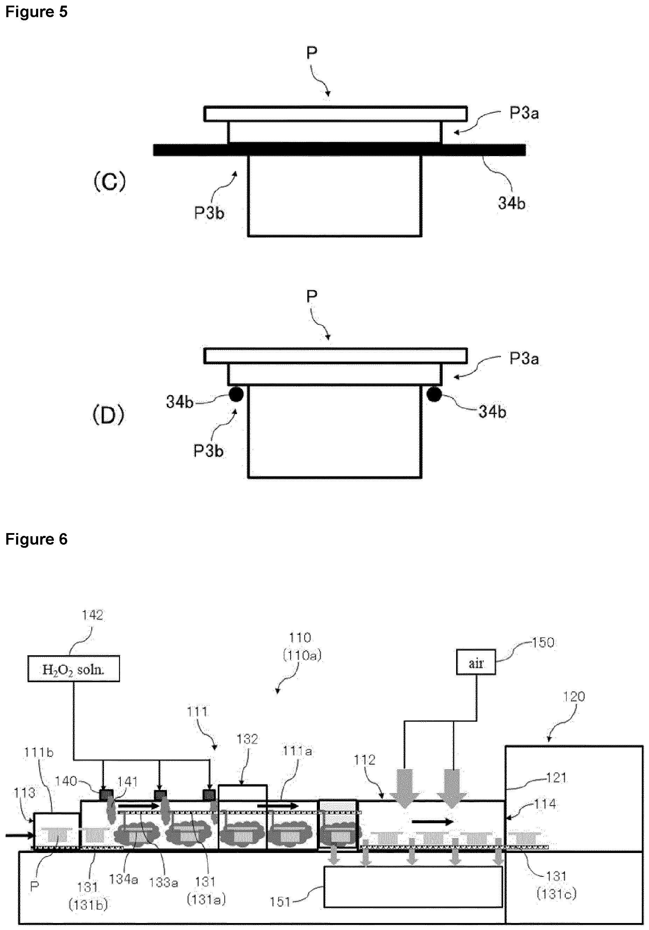

FIG. 5(C) is a front view showing the state where the support of the packages P is changed from the rolling conveyor 31a to the rolling conveyor 31b by the pusher 32, and FIG. 5(D) is a side view thereof. FIG. 5 illustrates a support bar 34b only and no drive shaft 33b. In FIG. 5, the support bar 34b supports a second shoulder portion P3b of the package P on both side surfaces.

Herein, the reason for changing a part for allowing the support bar to support the packages P from the first shoulder portion P3a to the second shoulder portion P3b will be described. The package P in contact with the rolling conveyor 31a has the first shoulder portion P3a that is in contact with the support bar 34a. The resulting insufficient contact with a hydrogen peroxide solution mist during a decontamination process achieves no uniform condensation of a hydrogen peroxide thin film, thereby reducing decontamination effects. Then, all external surfaces of the packages P can completely be decontaminated by changing the support portion from the first shoulder portion P3a to the second shoulder portion P3b.

Subsequently, the packages P supported at the top portion of the rolling conveyor 31b are conveyed in the lowered direction (from the top portion to the bottom portion) inside the decontamination region third portion 11a (3) as the rolling conveyor 31b is operated in an intermittent manner. In this state, a uniform hydrogen peroxide thin film is condensed on the external surfaces of the packages P to be decontaminated. Accordingly, the packages P are uniformly decontaminated entirely on external surfaces thereof by staying for a predetermined period inside the decontamination region body 11a while being conveyed by the rolling conveyors 31a, 31b.

Subsequently, the packages P are conveyed in the lowered direction while they are supported by the rolling conveyor 31b, and carried to the inside of the aeration region body 12a. The packages P carried to the inside of the aeration region body 12a are conveyed in the lowered direction inside the aeration region body 12a for aeration. Specifically, an air supply device 50 of the aeration device supplies clean air to the inside of the aeration region body 12a. In addition, the air inside the aeration region body 12a (incl. evaporated hydrogen peroxide and hydrogen peroxide solution mist) is forcibly discharged by an air exhaust device (not shown) of the aeration device. Also, the hydrogen peroxide in the forcibly discharged air is resolved into oxygen and water by a hydrogen peroxide decomposition unit 51.

The air supply and exhaust amount of clean air and aeration time in the aeration operation are predetermined conditions. Accordingly, the packages P are conveyed inside the aeration region body 12a for aeration to remove the hydrogen peroxide thin film condensed on the surface and then completely decontaminated.

Subsequently, the packages P are placed from the rolling conveyor 31b to the roller conveyor 31d at the bottom portion of the aeration region body 12a. Herein, the rolling conveyor 31b and the roller conveyor 31d are alternately operated in an intermittent manner. Specifically, the rolling conveyor 31b is operated with the roller conveyor 31d stopped to convey and stop the packages P to the bottom portion of the roller conveyor 31b. Subsequently, the roller conveyor 31d is operated with the rolling conveyor 31b stopped, the packages P are removed from the support bar 34b of the rolling conveyor 31b and placed on the roller conveyor 31d, and carried to the inside of the isolator 20 through the outlet 14 of the delivery region 12b.

Accordingly, in the packages P carried to the inside of the isolator 20 after decontamination, the upper surface seal P2 is peeled open from the package P in the isolator 20 and the packages are filled with syringes or vials sterilized therein.

Subsequently, the mist supply device 40 will be described. In this first embodiment, the mist supply device 40 used is an ultrasonic atomizer 40. The ultrasonic atomizer 40 is disposed on a side wall surface of the decontamination region body 11a to discharge from the side surface a hydrogen peroxide solution mist 41 toward the packages P conveyed by the rolling conveyor 31a (see FIG. 3). A hydrogen peroxide solution is supplied from the hydrogen peroxide solution tank 42 disposed outside the decontamination region body 11a to the ultrasonic atomizer 40. The amount of the hydrogen peroxide solution supplied (consumed) can be controlled to properly decontaminate the external surfaces of the packages P.

The structure of the ultrasonic atomizer 40 is not particularly restricted. For example, an immersion-type ultrasonic atomizer for atomizing a hydrogen peroxide solution with a piezoelectric vibrator placed therein can be employed. The ultrasonic atomizer may be a disk mesh type atomizer including a piezoelectric vibrator and a perforated vibration plate provided with a plurality of micropores for atomizing a hydrogen peroxide solution by vibration of the piezoelectric vibrator, the micropores passing through the perforated vibration plate between the front surface and the back surface thereof. In this first embodiment, a disk mesh type atomizer employed supplies a hydrogen peroxide solution from one surface of a perforated vibration plate and supplies a hydrogen peroxide solution mist discharged from the other surface to the inside of the decontamination region body 11a.

The hydrogen peroxide solution mist generated by the ultrasonic atomizer 40 is converted into a fine particle containing mists, fogs and fine liquid droplets as described above and uniformly floats inside the decontamination region body 11a. Accordingly, a uniform and thin hydrogen peroxide solution film that is condensed entirely on the external surfaces of the packages P that move up and down inside the decontamination region body 11a is formed. The thin hydrogen peroxide solution film is subjected to repeated phase change of condensation and evaporation between a hydrogen peroxide solution and a hydrogen peroxide gas to provide advanced decontamination effects of the packages P.

Also, by repeated re-evaporation and condensation of the uniformly and thinly formed hydrogen peroxide solution film condensed entirely on the external surfaces of the packages P, the concentration of a hydrogen peroxide solution in a hydrogen peroxide solution mist can be increased and efficient decontamination can be performed with a small amount of hydrogen peroxide solution. Such an efficient decontamination with a small amount of hydrogen peroxide solution can improve the efficiency of aeration for the hydrogen peroxide solution film that is residual on the surface of the packages P and reduce the duration of decontamination operations.

Thus, the above first embodiment can provide a continuous decontamination device capable of achieving short-term operations to provide uniform decontamination levels at each part by employing decontamination agents such as hydrogen peroxide that have recently been widely used without using expensive electron accelerators and of treating articles to be decontaminated in large quantities.

### <Second embodiment>

This second embodiment relates to a horizontal continuous decontamination device while the above first embodiment relates to a vertical continuous decontamination device. An accommodated body (package) to be decontaminated is the same package P as in the above first embodiment.

The continuous decontamination device of this second embodiment will be described. FIG. 6 is a schematic cross-sectional view showing the inside of a continuous decontamination device according to the second embodiment seen in a front view. The continuous decontamination device according to this second embodiment, as in the above first embodiment, includes a device body composed of a decontamination region and an aeration region, a conveyance device for conveying a package P, a mist supply device, and an aeration device (an air supply and exhaust device of clean air for aeration). The continuous decontamination device according to this second embodiment is a horizontal continuous decontamination device for conveying a package P in the horizontal direction in the decontamination region and the aeration region.

In FIG. 6, a device body 110a of a horizontal continuous decontamination device 110 according to this second embodiment is covered with an outer wall portion made of stainless metal plate on the periphery, linked to a side wall 121 of an isolator 120, and placed on the floor. The device body 110a is divided into a decontamination region 111 and an aeration region 112. The decontamination region 111 is divided into a decontamination region body 111a and an introduction region 111b. One wall portion of the introduction region 111b is provided with an inlet 113 for carrying a package P to the inside of the continuous decontamination device 110. Meanwhile, one wall portion of the aeration region 112 is provided with an outlet 114 for carrying a package P out of the device, which communicates with the side wall 121 of the isolator 120.

A conveyance device 130 for conveying a package P is disposed inside the device body 110a. The conveyance device 130 is configured by an article conveyance device 131 and a support changing device 132. The article conveyance device 131 includes a hanging conveyor 131a for conveying a package P in the horizontal direction inside the decontamination region body 111a, a roller conveyor 131b for conveying the package P in the horizontal direction from the inlet 113 of the introduction region 111b to an introduction portion of the decontamination region body 111a, and a roller conveyor 131c for conveying the package P in the horizontal direction from the inside of the aeration region 112 to the outlet 114. Meanwhile, the support changing device 132, which is provided in a middle area of the decontamination region body 111a, removes the package P from the hanging conveyor 131a and changes the support portion to allow the hanging conveyor 131a to support the package again.

The type of a conveyance device for conveying a package P is not particularly restricted. Such a conveyance device may be a combination of a roller conveyor for conveying an article on which a bottom wall surface thereof is placed, a conveyor device such as a mesh conveyor, and a support device such as a timing belt for conveying by supporting a side surface portion, a timing lift, a rolling conveyor and a shuttle conveyor.

Herein, the structure and operation of a hanging conveyor employed in this second embodiment will be described. In FIG. 6, the hanging conveyor 131a includes 2 sets of carriers having the same structure for supporting a package P on both side surfaces. FIG. 6 illustrates one carrier only and no other carrier hidden on its back side.

Each of the carriers is configured by two drive shafts 133a and a plurality of support bars 134a arranged under these drive shafts (see FIG. 6). The support bars 134a are each an L-shaped bar, which is hung below the drive shaft 133a. The two drive shafts 133a are each composed of a loop-shaped chain disposed in the horizontal direction at the decontamination region body 111a, which is revolved in the horizontal direction (in the right-and-left direction in the figure) by a drive mechanism (not shown). Thus, as the two drive shafts 133a are revolved in the horizontal direction, the plurality of support bars 134a is revolved along the shafts in the horizontal direction. In rotation, the plurality of support bars 134a conveys the package P in the horizontal direction (in the right-and-left direction in the figure) inside the decontamination region body 111a by supporting the package P on both side surfaces.

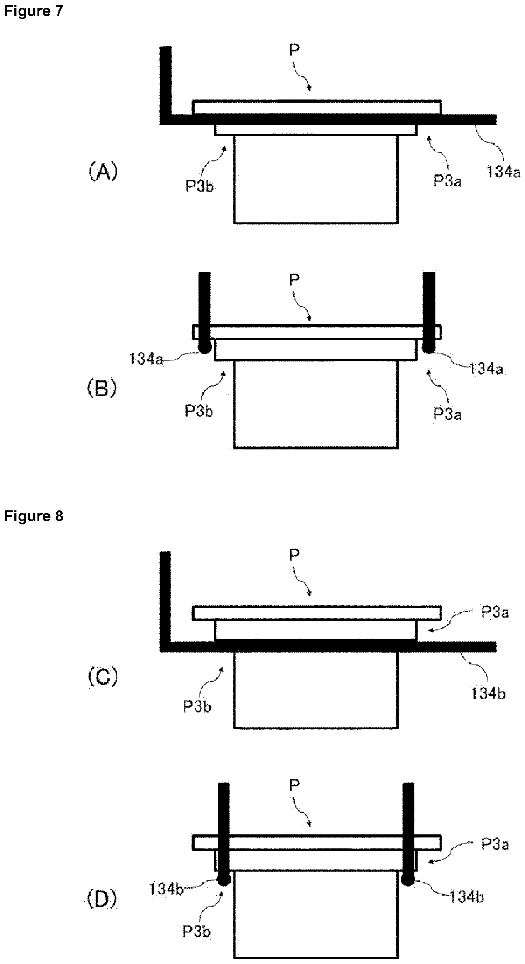

Subsequently, the state where the support bar 134a supports the package P on both side surfaces will be described. FIG. 7(A) is a front view showing the state where the hanging conveyor 131a of this second embodiment supports the package P, and FIG. 7(B) is a side view thereof. FIG. 7 illustrates a support bar 134a only and no drive shaft 133a. In FIG. 7, the support bar 134a supports a first shoulder portion P3a of the package P on both side surfaces. In this state, the packages P are conveyed by the hanging conveyor 131a in the horizontal direction inside the decontamination region body 111a (see FIG. 6). The package P includes a second shoulder portion P3b below the first shoulder portion P3a. The operation of the second shoulder portion P3b will be described later.

In such a configuration, the inside of the continuous decontamination device 110 and decontamination operations will be described. In FIG. 6, an operator (not shown) who stays in the external environment places a plurality of packages P on the roller conveyor 131b for driving. The packages P are carried to the inside of the introduction region 111b through the inlet 113 of the introduction region 111b, while being placed on the roller conveyor 131b for driving.

Subsequently, the packages P are carried to the inside of the decontamination region body 111a and the support of the packages P is changed from the roller conveyor 131b to the hanging conveyor 131a. The support changing operation from the roller conveyor 131b to the hanging conveyor 131a is alternately performed in an intermittent manner by the roller conveyor 131b and the hanging conveyor 131a as in the above first embodiment. The packages P supported by the hanging conveyor 131a are conveyed in the horizontal direction inside the decontamination region body 111a. A plurality of mist supply devices 140 (3 devices in FIG. 6) is disposed on an upper wall surface of the decontamination region body 111a to discharge from the upper surface a hydrogen peroxide solution mist 141 toward the packages P conveyed by the hanging conveyor 131a.

As a result, the hydrogen peroxide solution mist 141 is uniformly filled inside the decontamination region body 111a to continuously decontaminate the plurality of packages P being conveyed. The mist supply device 140 will be described later. Accordingly, the packages P are uniformly decontaminated entirely on external surfaces thereof by staying for a predetermined period inside the decontamination region body 111a while being conveyed by the hanging conveyor 131a.

Accordingly, the support of the packages P decontaminated inside the decontamination region body 111a is changed by the hanging conveyor 131a as the support changing device 132 is operated in the middle area of the decontamination region body 111a. Herein, the structure of the support changing device 132 is not restricted at all. In this second embodiment, the same pusher 132 as in the above first embodiment is used. FIG. 6 shows a linear traveling direction of the decontamination region body 111a. However, the traveling direction of the decontamination region body 111a is not restricted to that, and it may be changed, depending on the position of the support changing device 132. For example, a 90° rotation of the packages can make the decontamination region body 111a horizontal L-shaped, thereby providing solutions of restrictions on the location of the horizontal continuous decontamination device 110.

FIG. 8(C) is a front view showing the state where the support of the packages P is changed on the hanging conveyor 131a, and FIG. 8(D) is a side view thereof. FIG. 8 illustrates a support bar 134b only and no drive shaft 133b. In FIG. 8, the support bar 134b supports a second shoulder portion P3b of the package P on both side surfaces. The reason for changing a part for allowing the support bar to support the packages P from the first shoulder portion P3a to the second shoulder portion P3b is the same as in the above first embodiment.

Subsequently, the packages P that have reached an end portion of the decontamination region body 111a are carried to the inside of the aeration region body 112a and the support is changed from the hanging conveyor 131a to the roller conveyor 131c. In this state, a hydrogen peroxide thin film is still condensed on external surfaces of the packages P conveyed to the introduction portion of the aeration region 112 by the hanging conveyor 131a.

Subsequently, the packages P carried to the inside of the aeration region 112 are conveyed in the horizontal direction inside the aeration region 112 for aeration. Specifically, an air supply device 150 of the aeration device supplies clean air to the inside of the aeration region 112. In addition, the air inside the aeration region 112 (incl. evaporated hydrogen peroxide and hydrogen peroxide solution mist) is forcibly discharged by an air exhaust device (not shown) of the aeration device. Also, the hydrogen peroxide in the forcibly discharged air is resolved into oxygen and water by a hydrogen peroxide decomposition unit 151.

The air supply and exhaust amount of clean air and aeration time in the aeration operation are predetermined conditions. Accordingly, the packages P are conveyed inside the aeration region 112 for aeration to remove the hydrogen peroxide thin film condensed on the surface and then completely decontaminated.

Subsequently, the packages P are carried to the inside of the isolator 120 through the outlet 114 of the aeration region 112, while being placed on the roller conveyor 131c.

Accordingly, in the packages P carried to the inside of the isolator 120 after decontamination, the upper surface seal P2 is peeled open from the package P in the isolator 120 and the packages are filled with syringes or vials sterilized therein.

Subsequently, the mist supply device 140 will be described. In this second embodiment, the mist supply device 140 used is the same ultrasonic atomizer 140 as in the above first embodiment. The ultrasonic atomizer 140 is disposed on an upper wall surface of the decontamination region body 111a to discharge from the upper surface the hydrogen peroxide solution mist 141 toward the packages P conveyed by the hanging conveyor 131a (see FIG. 6). A hydrogen peroxide solution is supplied from the hydrogen peroxide solution tank 142 disposed outside the decontamination region body 111a to the ultrasonic atomizer 140. The amount of the hydrogen peroxide solution supplied (consumed) can be controlled to properly decontaminate the external surfaces of the packages P.

The structure of the ultrasonic atomizer 140 is not particularly restricted. In this second embodiment, the same disk mesh type atomizer as in the above first embodiment is employed.

The hydrogen peroxide solution mist generated by the ultrasonic atomizer 140 is converted into a fine particle containing mists, fogs and fine liquid droplets as described above and uniformly floats inside the decontamination region body 111a. Accordingly, a uniform and thin hydrogen peroxide solution film that is condensed entirely on the external surfaces of the packages P that move horizontally inside the decontamination region body 111a is formed. The thin hydrogen peroxide solution film is subjected to repeated phase change of condensation and evaporation between a hydrogen peroxide solution and a hydrogen peroxide gas to provide advanced decontamination effects of the packages P.

Also, by repeated re-evaporation and condensation of the uniformly and thinly formed hydrogen peroxide solution film condensed entirely on the external surfaces of the packages P, the concentration of a hydrogen peroxide solution in a hydrogen peroxide solution mist can be increased and efficient decontamination can be performed with a small amount of hydrogen peroxide solution. Such an efficient decontamination with a small amount of hydrogen peroxide solution can improve the efficiency of aeration for the hydrogen peroxide solution film that is residual on the surface of the package P and reduce the duration of decontamination operations.

Thus, the above second embodiment can provide a continuous decontamination device capable of achieving short-term operations to provide uniform decontamination levels at each part by employing decontamination agents such as hydrogen peroxide that have recently been widely used without using expensive electron accelerators and of treating articles to be decontaminated in large quantities.

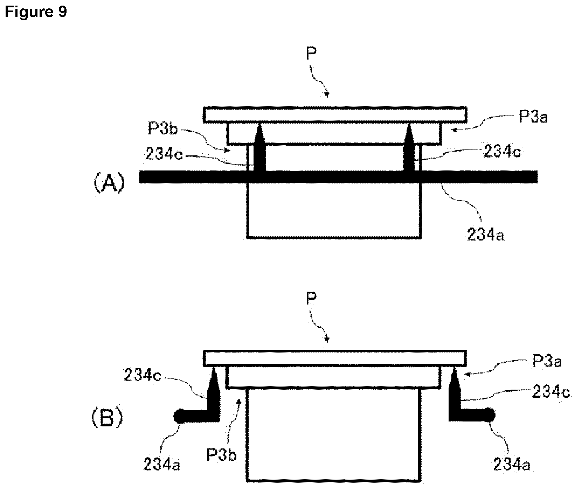

The present invention is achieved by not only each of the above embodiments, but also by the following various alternatives.
(1) In each of the above embodiments, a package P is supported by a support bar on a rolling conveyor or a hanging conveyor. However, the configuration is not restricted to that, and a package P may be supported by a support catch in place of a support bar. The state is shown in FIG. 9. In FIG. 9, a shoulder portion of the package P is supported by two support catches at the edge. Accordingly, reductions on the decontamination level can be prevented even at a support portion (at point).
(2) In each of the above embodiments, the mechanism for changing the support of the package P used is a pusher. However, the configuration is not restricted to that, and a gripper may be employed to change the support to grip the package P with a gripper. When a gripper is employed, the support can be changed by rotating the package P by 90°.
(3) In each of the above embodiments, the support of the package P is changed by a pusher in the middle of a decontamination region body. However, the configuration is not restricted to that, and a mechanism of alternately changing the support by one step by a rolling conveyor that rises as it is operated in an intermittent manner may be employed. In addition, a mechanism of alternately changing the support by one step by a hanging conveyor may be employed.
(4) In each of the above embodiments, the support of the package P is changed at between a first shoulder portion and a second shoulder portion on the same side surface of the package P. However, the configuration is not restricted to that, and the support of the package P may be changed at between a first shoulder portion or a second shoulder portion on other surface of the package P.
(5) In each of the above embodiments, the support of the package P is changed at between a first shoulder portion and a second shoulder portion on the same side surface of the package P. However, the configuration is not restricted to that, and the support of the package P may be changed at between a shoulder portion and a bottom surface portion of the package P.

### REFERENCE SIGNS LIST

10, 110...Continuous decontamination device, 10a,
110a...Device body, 11, 111...Decontamination region, 11a (11a (1), 11a (2), 11a (3)), 111a...Decontamination region body,
11b, 111b...Introduction region, 12, 112...Aeration region,
12a, 112a...Aeration region body, 12b...Delivery region,
13, 113...Inlet, 14, 114...Outlet,
20, 120...Isolator, 21, 121...Side wall, 30, 130...Conveyance device,
31a, 31b...Rolling conveyor, 131a...Hanging conveyor,
31c, 31d, 131b, 131c...Roller conveyor,
32, 132...Support changing device (Pusher), 32a...Cylinder,
33a, 33b, 133a, 133b...Drive shaft,
34a, 34b, 134a, 134b, 234a...Support bar,
234c...Support catch,
40, 140...Mist discharging device, 41, 141...Hydrogen peroxide solution mist,
42, 142...Hydrogen peroxide solution tank, 50, 150...Air supply device,
51, 151...Hydrogen peroxide decomposition unit, P...Package, Pl...tab,
P2...Upper surface seal, P3...Side surface shoulder portion, P3a...First shoulder portion, P3b...Second shoulder portion.

## Claims

1. A continuous decontamination device connected to a sterile working chamber for decontaminating external surfaces of an article with a decontamination agent mist and conveying the article to the inside of the sterile working chamber, the continuous decontamination device comprising:
a device body composed of a decontamination region and an aeration region; a conveyance means for conveying an article; a mist supply means; and an aeration means, wherein
the device body includes an inlet for carrying the article before decontamination in the decontamination region and an outlet for carrying the decontaminated article out of the aeration region,
the conveyance means can decontaminate all external surfaces of the article by changing a part for supporting the article inside the decontamination region when the conveyance means supports the article carried from the inlet and conveys the article to the outlet through the inside of the decontamination region and the aeration region,
the mist supply means includes an ultrasonic atomizer for converting a decontamination agent into the decontamination agent mist and supplying the same to the inside of the decontamination region to concentrate the decontamination agent mist on external surfaces of the article conveyed inside the decontamination region by the conveyance means, and
the aeration means removes with clean gas the decontamination agent mist that is residual on the external surfaces of the article conveyed by the conveyance means from the decontamination region.

2. The continuous decontamination device according to claim 1, wherein
the conveyance means includes an article conveyance device for conveying an article inside a decontamination region and an aeration region and a support changing device for changing a part for supporting the article, wherein
the article conveyance device continuously conveys a plurality of articles carried from the inlet in either an elevated or a lowered direction inside the decontamination region and the aeration region to carry the articles out of the outlet, and
the support changing device removes the articles from the article conveyance device inside the decontamination region and supports the articles on the article conveyance device again.

3. The continuous decontamination device according to claim 1, wherein
the conveyance means includes an article conveyance device for conveying an article inside a decontamination region and an aeration region and a support changing device for changing a part for supporting the article, wherein
the article conveyance device continuously conveys a plurality of articles carried from the inlet in the horizontal direction inside the decontamination region and the aeration region to carry the articles out of the outlet, and
the support changing device removes the articles from the article conveyance device inside the decontamination region and supports the articles on the article conveyance device again.

4. The continuous decontamination device according to any one of claims 1 to 3, wherein
the article to be decontaminated is an accommodated body for accommodating a medical appliance such as sterile syringes and vials.
